# EUROPÄISCHE PATENTSCHRIFT

(11) **EP 2 563 302 B1**
(45) Veröffentlichungstag und Bekanntmachung des Hinweises auf die Patenterteilung: **04.01.2017**
(21) Anmeldenummer: 11714969.0
(22) Anmeldetag: 13.04.2011
(51) Int. Cl.: A61F 7/08, G01K 11/12

(54) **WÄRMFLASCHE AUS KUNSTSTOFF**
HOT-WATER BOTTLE MADE OF PLASTIC
BOUILLOTTE EN PLASTIQUE

(30) Priorität: 21.05.2010 DE 202010007284 U; 29.04.2010 DE 202010006479 U
(43) Veröffentlichungstag der Anmeldung: 06.03.2013
(73) Patentinhaber: Fashy GmbH Produktion und Vertrieb, 70825 Korntal-Münchingen (DE)
(72) Erfinder: KRAUS, Alexander, 71229 Leonberg (DE)
(74) Vertreter: DREISS Patentanwälte PartG mbB
(86) Internationale Anmeldenummer: PCT/EP2011/055783
(87) Internationale Veröffentlichungsnummer: WO 2011/134793

(56) Entgegenhaltungen:
- WO-A1-2004/042299
- DE-U1-202004 009 808
- DE-U1-202005 006 301
- GB-A- 2 257 367
- JP-A- 2005 083 933
- US-A1- 2006 081 639

## Beschreibung

Die vorliegende Erfindung bezieht sich auf eine Wärmflasche aus Kunststoff nach dem Oberbegriff des Anspruchs 1.

Bei einer derartigen aus der DE 20 2005 006 301 U1 bekannten Wärmflasche aus Kunststoff ist ein Temperaturindikator durch temperaturabhängig aktivierbare Farbbereiche ausgebildet, die auf der Außenseite des Flaschenkorpus angeordnet sind. Damit ist lediglich erreicht, dass die Temperatur des flüssigen Mediums innerhalb der Wärmflasche visuell wahrgenommen und entschieden werden kann, ob der Wärmflaschenkorpus bzw. das in der Wärmflasche enthaltene flüssige Medium für den Benutzer den richtigen bzw. ertragbaren Temperaturwert aufweist. Damit ist der angezeigte Temperaturbereich nur ein Anhaltswert. Aus der GB 2 257 367 A und der DE 20 2005 006301 U sind Wärmflaschen aus Kunststoff mit einem Überhitzungsindikator für das einzufüllende Medium bekannt, der durch einen Innen- oder Außenbereich der Wandung gebildet wird.
Aus der WO 2004/042299 A1 ist eine Vorrichtung zur Temperaturüberwachung in einem Kühlschrank bekannt, die einen temperaturemfindlichen Schwimmkörper umfasst. Dieser Schwimmkörper ist von einem thermischen Puffermasse umgeben, so dass er Temperaturänderungen zeitverzögert anzeigt.

Wärmflaschen aus Kunststoff sollten jedoch nicht mit einem flüssigen Medium über einen bestimmten Temperaturwert hinaus, also beispielsweise nicht mit kochendem Wasser, befüllt werden, da das zu heiße flüssige Medium und insbesondere auch der bei zu heißem Medium sich ergebende Mediumdampf den bei Wärmflaschen verwendeten Kunststoff nachteilig beeinflussen können. Außerdem besteht für den Nutzer die Gefahr von Verbrennungen.

Aufgabe der vorliegenden Erfindung ist es deshalb, eine Wärmflasche aus Kunststoff mit einem Überhitzungsindikator zu schaffen, der die unmittelbar auf das Kunststoffmaterial einwirkende Temperatur des flüssigen Mediums optisch anzeigt, woraus eine optisch wahrnehmbare Warnung der Überhitzung resultiert.

Zur Lösung dieser Aufgabe sind bei einer Wärmflasche aus Kunststoff der genannten Art die im Anspruch 1 angegebenen Merkmale vorgesehen.

Durch die erfindungsgemäßen Maßnahmen ist bei jeder der alternativen Möglichkeiten erreicht, dass der Überhitzungsindikator mit dem flüssigen Medium beziehungsweise dem flüssigen Mediumdampf unmittelbar in Verbindung kommt und damit eine genaue und unmittelbare optische Überhitzungsanzeige gewährleistet.

Gemäß den in einem oder mehreren der Ansprüche 2 bis 4 beschriebenen Ausführungsbeispielen ist der Überhitzungsindikator durch ein thermochromes Material beziehungsweise Substanz gebildet, das beziehungsweise die die Eigenschaft besitzt, bei Erwärmung die Farbe zu ändern und nach Abkühlen reversibel wieder die ursprüngliche Farbe einzunehmen.

Weitere vorteilhafte Ausgestaltungen hinsichtlich der Orte an der Wärmflasche, die mit dem flüssigen Medium oder deren Dampf unmittelbar in Verbindung gelangen, sind durch die Merkmale eines oder mehrerer der Ansprüche 5 und 7 bis 10 verwirklicht.

Mit den Merkmalen des Anspruchs 6 ist erreicht, dass ausgehend von einer Überhitzungstemperatur mit dem Abkühlen des flüssigen Mediums eine visuelle Wahrnehmbarkeit anderer komfortabler Temperaturbereiche möglich ist.

In vorteilhafter Weise wird gemäß den Merkmalen des Anspruchs 11 der Überhitzungsindikator auf eine bestimmte Temperatur eingestellt, um eine nachteilige Beeinflussung des für die Wärmflasche verwendeten Kunststoffs zu vermeiden und um den Nutzer Sicherheit vor Verbrennungen o. dgl. zu bieten.

Weitere Einzelheiten der Erfindung sind der folgenden Beschreibung zu entnehmen, in der die Erfindung anhand der in der Zeichnung dargestellten Ausführungsbeispiele näher beschrieben und erläutert ist. Es zeigen:
Figur 1 in schematischer Vorderansicht eine Wärmflasche aus Kunststoff mit einem Überhitzungsindikator gemäß einem ersten und einem zweiten Ausführungsbeispiel vorliegender Erfindung und
Figur 2 einen Schnitt längs der Linie II-II der Figur 1 gemäß dem ersten und zweiten Ausführungsbeispiel sowie in strich-punktierten Linien gemäß weiteren Ausführungsbeispielen.

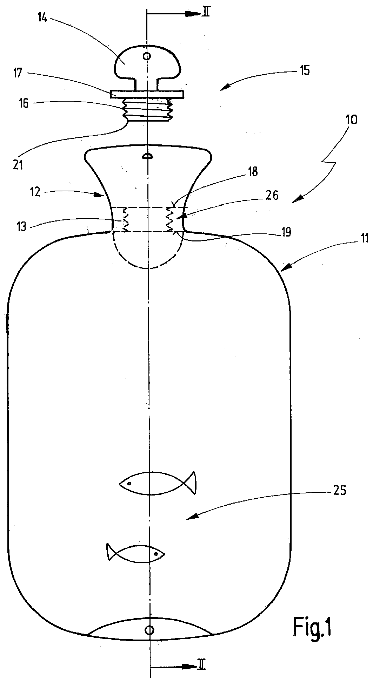

Figur 1 zeigt eine in üblicher Weise ausgestaltete Wärmflasche 10 aus Kunststoff, wie bspw. PVC, die einen Flaschenkorpus 11, einen in Form eines Einfülltrichters ausgebildeten Flaschenhals 12 sowie einen im Flaschenhals 12 ortsfest angeordneten, beispielsweise eingespritzten Innengewindering 13 in Form einer Innengewindebüchse für einen Verschluss 15 in Form eines Schraubstopfens aufweist. Der Flaschenhals 12 ist mit dem Flaschenkorpus 11 einstückig und im Wesentlichen aus demselben Kunststoffmaterial. Der Innengewindering 13 ist derart innerhalb des Flaschenhalses 12 gehalten, dass seine axial äußere Ringfläche 18 sowie seine axial innere Ringfläche 19 freiliegen. In das Innengewinde des Innengewinderings 13 ist der Verschluss 15 mit seinem zylindrischen Außengewindeteil 16 einschraubbar, wobei eine den Außengewindeteil 16 einstückig haltende Dichtplatte 17 an der axial äußeren Ringfläche 18 in eingeschraubten Zustand flüssigkeits- und dampfdicht anliegt. Dem Außengewindeteil 16 abgewandt ist an der Dichtplatte 17 eine hier plattenförmige Handhabe 14 bzw. Griffteil einstückig angebracht.

Die Wärmflasche 10 besitzt gemäß verschiedener Ausführungsbeispiele jeweils einen oder mehrere der in der Zeichnung dargestellten Überhitzungsindikatoren 25, 26, 27, 28 beziehungsweise 29. Jeder dieser Überhitzungsindikatoren 25 bis 29 besitzt ein thermochromes Material aus einer oder mehreren bestimmten Substanzen, die bei Erwärmung die Farbe ändern und bei Abkühlung reversibel ihre Ausgangsfarbe wieder annehmen. Bei den Ausführungsbeispielen sind diese thermochromen Materialien beispielsweise auf eine Temperatur im Bereich von etwa 60°C bis 80°C vorzugsweise bei etwa 80°C eingestellt, das heißt, dass die Indikatoren bei Erreichen und Überschreiten dieser Temperatur ihre Farbe verändern und so dem Nutzer visuell wahrnehmbar anzeigen, dass auf Grund des zu heißen flüssigen Mediums, wie beispielsweise Wasser, eine Überhitzung der Wärmflasche 10 erfolgt ist, die den Nachteil mit sich bringen kann, dass das für die Wärmflasche 10 verwendete Kunststoffmaterial oder -materialien nachteilig beeinflusst werden kann bzw. können und der Nutzer Verbrennungen o.dgl. davontragen kann.

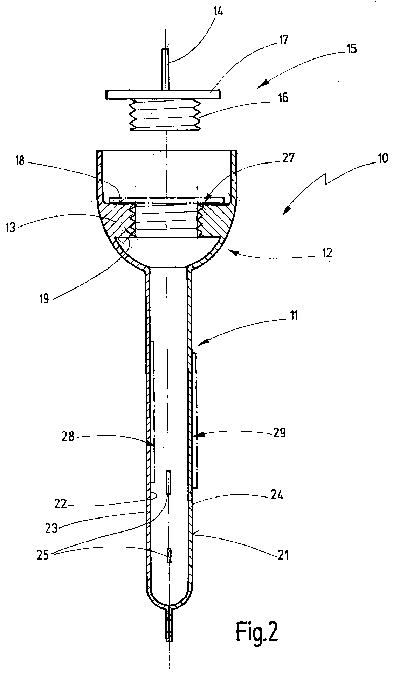

Bei dem in Figur 1 und in Figur 2 in ausgezogenen Linien dargestellten Überhitzungsindikator 25 sind ein oder mehrere schwimmfähige Elemente, hier in Form von Fischen, innerhalb des Flaschenkorpus 11 angeordnet, die einen thermochromen Kunststoff aufweisen und die in dem flüssigen Medium, wie beispielsweise Wasser, schwimmen. Dabei ist der verwendete Kunststoff, wie PE oder PP, mit thermochromen Masterbatches bzw. Farbgranulate versetzt, so dass der oder die Fische 25 insgesamt bei einer Temperatur von beispielsweise etwa 80°C ihre Farbe verändern.

Dieses Ausführungsbeispiel ist insbesondere bei Wärmflaschen 10 anwendbar und von Vorteil, die aus einem transparenten Kunststoff hergestellt sind, wobei es auch ausreichend sein kann, nur einen Teil oder eine Seite des Flaschenkorpus 11 aus diesem transparenten Kunststoff herzustellen. Ein weiterer Vorteil dieses Ausführungsbeispiels ist darin zu sehen, dass der visuelle Hinweis auf eine Überhitzung der Wärmflasche 10 schon in einem anfänglichen Befüllzustand, also noch bevor die Wärmflasche 10 im Wesentlichen vollständig befüllt ist, erscheint.

Bei einer Variante des Ausführungsbeispieles nach Figur 1 sind mehrere, beispielsweise drei schwimmfähige Elemente bspw. in Form der Fische 25 im Innenraum des Flaschenkorpus 11 der Wärmflasche 10 untergebracht. Alle drei Fische 25 sind derart unterschiedlich mit thermochromen Masterbatches bzw. Farbgranulaten versetzt, dass die Fische 25 in kaltem bzw. in Umgebungstemperaturzustand ihre Ausgangsfarbe wie bspw. blau, grün bzw. rot aufweisen. Die Fische 25 sind außerdem thermochrom so eingestellt, dass jeder dieser Fische bei einer anderen Temperatur bzw. in einem anderem Temperaturbereich bspw. nach weiß verfärbt. So ist der "rote" Fisch thermochrom so eingestellt, dass er sich bei der Überhitzungstemperatur im Bereich von etwa 80°C nach weiß verfärbt, während sich der "grüne" Fisch bei einer Temperatur im Bereich von 60° bis 65°C und der "blaue" Fisch bei einem Temperaturbereich von etwa 40° bis 45°C nach weiß verfärbt.

Mit anderen Worten, ist mit dem Einfüllen des flüssigen heißen Mediums eine Temperatur im Bereich von ≥ 80°C erreicht, werden alle drei Fische 25 sich nach weiß verfärben, was anzeigt, dass Gefahr für das Wärmflaschenmaterial gegeben ist. Sind demnach alle drei Fische 25 nach weiß verfärbt, stellt dies den Überhitzungsindikator dar. Wird versucht, das zu heiße flüssige Medium abzukühlen, wird der Überhitzungsindikator in Form des "roten" Fisches sich unterhalb von 80°C wieder in seine Ausgangsfarbe wandeln, was dann auf eine Temperatur des flüssigen Mediums in der Wärmflasche 10 im Bereich von 60/65°C bis < 80°C hinweist. Wenn dann bei weiterem Abkühlen der "grüne" Fisch sich ebenfalls in seine ursprüngliche Farbe verwandelt, bedeutet dies sozusagen eine Wohlfühltemperatur des flüssigen Mediums innerhalb der Wärmflasche 10 in einem Bereich von 40/45°C bis unterhalb 60°C. Entsprechendes gilt bei weiterem Abkühlen des flüssigen Mediums.

Damit kann neben der Überhitzungsanzeige auch eine visuelle Wahrnehmung weiterer Temperaturwerte bzw. Temperaturbereiche ermöglicht sein. Es versteht sich, dass andersartig und/oder unterschiedlich geformte schwimmfähige Elemente 25 Verwendung finden können.

Gemäß einem zweiten Ausführungsbeispiel ist der Innengewindering 13, der aus einem anderen Kunststoff als der Wärmflaschenkorpus 11 hergestellt ist, nämlich bspw. wie Einlegeelemente 25 aus PE oder PP, ebenfalls mit thermochromen Maserbatches bzw. Farbgranulaten versehen, so dass dadurch ein Überhitzungsindikator 26 gebildet ist, der auch bei nicht transparenten Wärmflaschen 10 bzw. Flaschenkorpus 11 eingesetzt werden kann, da hier die Farbveränderung auf die Ringfläche 18 und am Innengewindebereich sichtbar ist.

In Figur 2 sind weitere Ausführungsbeispiele der Überhitzungsindikatoren 26 bis 28 schematisch und strichpunktiert dargestellt.

Der Überhitzungsindikator 27 ist durch eine Ringfolie gebildet, die vorzugsweise an der axial äußeren Ringfläche 18 des Innengewinderings 13 angebracht ist. Diese Indikator-Ringfolie 26 aus thermochromem Kunststoff wird durch Berührung mit der zu heiß eingefüllten Flüssigkeit (Wasser) auf Temperatur gebracht und ändert die Farbe mit Erreichen der vorgenannten Überhitzungstemperatur. Eine derartige Indikator-Ringfolie 27 kann zusätzlich oder alternativ auf die axial innere Ringfläche 19 des Innengewinderings 13 aufgebracht werden.

Diese Ausgestaltung(en) 27 und die weiter oben genannte Ausgestaltungen 25 und 26 können auch bei Wärmflaschen 10 aus durchscheinendem (transluzentem) Kunststoff Verwendung finden.

Weitere Ausführungsbeispiele von Überhitzungsindikatoren 28 und 29 sind durch eine Beschichtung eines Bereichs der Innenfläche 22 und/oder der Außenfläche 21 einer der beiden oder beider Wandungen 23, 24 des Flaschenkorpus 11 mit einem thermochromen Material gegeben. Diese Indikatorbeschichtung 28 bzw. 29 kann über eine größere oder kleinere Fläche der Innenfläche 22 bzw. Außenfläche 21 der Wandung 23, 24 erfolgen, wobei im Falle der Innenfläche dies nicht nur bei transparentem sondern auch transluzentem Kunststoff des Flaschenkorpus 11 angewendet werden, insbesondere durch die Wahl einer kontrastierenden Farbe, in die sich das verwendete thermochrome Material ändert, während im Falle der Außenfläche 21 auch opake Kunststoffe in Frage kommen. Die Indikatorbeschichtung 28 bzw. 29 kann bspw. durch einen thermochromen Lack gegeben sein, der auf den betreffenden Bereich der Innenfläche 22 der Wandung 23, 24 aufgesprüht wird, oder durch eine thermochrome Folie, die beim Herstellen des Flaschenkorpus 11 in eine Blas- oder Spritzgussform eingelegt wird. Der auf der Außenseite 21 der Wandung 23, 24 aufgebrachte Überhitzungsindikator 29 ist für die Farbänderung auf bspw. etwa 60°C eingestellt, um die thermische Isolierwirkung der Wandung 23, 24 zu berücksichtigen.

Es versteht sich, dass ein thermochromer Lack auch bei den Indikatorfischen 25, die dann aus einem üblichen Kunststoff mit aufgesprühtem oder durch Eintauchen aufgebrachten thermochromen Lack bestehen, eingesetzt werden kann und dass statt der Indikatorfolien 26 und 28 und 29 die betreffende Fläche(n) mit einem solchen thermochromen Lack versehen werden kann/können.

Sowohl der Kunststoff (bspw. PE oder PP) der Folie als auch der Lack können mit thermochromen Masterbatches bzw. Farbgranulaten versetzt sein.

In allen Ausführungsbeispielen reagiert der Überhitzungsindikator beim bzw. während des Einfüllens des flüssigen Mediums Wasser.

## Patentansprüche

1. Wärmflasche (10) aus Kunststoff, mit einem Flaschenkorpus (11), mit einem Flaschenhals (12), der einen Einfülltrichter bildet, mit einem Verschluss (15) zum dichtenden Einschrauben in einen Gewindeeinsatz (13) des Flaschenhalses (12) und mit einem Temperaturindikator in Form eines Überhitzungsindikators (25-29) für das einzufüllende beziehungsweise eingefüllte flüssige Medium, vorzugsweise Wasser, **dadurch gekennzeichnet, dass** der Temperaturindikator durch mindestens ein im flüssigen Medium des Flaschenkorpus (11) schwimmfähiges Einlegeteil aus oder mit einem thermochromen Kunststoff oder durch einen Bereich des Gewindeeinsatzes (13) im Flaschenhals (12), der an seiner axial äußeren und/oder inneren freiliegenden Ringstirn (18, 19) mit einer thermochromen Kunststofffolie belegt oder mit einem thermochromen Lack an einem freiliegenden Bereich versehen ist, gebildet ist.

2. Wärmflasche nach Anspruch 1, **dadurch gekennzeichnet, dass** das schwimmfähige Einlegeteil (25) und/oder der Gewindeeinsatz (13) aus einem Kunststoff hergestellt ist, der mit thermochromen Masterbatches versehen ist.

3. Wärmflasche nach Anspruch 1 oder 2, **dadurch gekennzeichnet, dass** mehrere schwimmfähige Einlegeteile (25) vorgesehen sind, von denen mindestens eines den Überhitzungsindikator bildet und von denen eines oder mehrere auf geringere Temperaturwerte thermochrom eingestellt sind.

4. Wärmflasche nach einem der Ansprüche 1 bis 3, **dadurch gekennzeichnet, dass** der Kunststoff, der Folie bzw. der Lack mit thermochromen Masterbatches versehen ist.

5. Wärmflasche nach mindestens einem der vorhergehenden Ansprüche, **dadurch gekennzeichnet, dass** der Überhitzungsindikator (25 - 29) auf eine bestimmte Temperatur im Bereich von 60°C bis 80°C, vorzugsweise bei etwa 80°C eingestellt ist.

## Claims

1. A hot-water bottle (10) made of plastic, with a bottle body (11), with a bottleneck (12) which forms a funnel tube, with a seal (15) for sealingly screwing into a threaded insert (13) of the bottleneck (12) and with a temperature indicator in the form of an overheating indicator (25-29) for the liquid medium, preferably water, which is to be or has been poured in, **characterized in that** the temperature indicator is formed by at least one floatable insert in the liquid medium of the bottle body (11), which insert is made of or with a thermochromic plastic, or is formed by an area of the threaded insert (13) in the bottleneck (12) which, at its axially outer and/or inner exposed ring front end (18, 19) is lined with a thermochromic plastic film or is provided with a thermochromic finish at an exposed area.

2. The hot-water bottle according to claim 1, **characterized in that** the floatable insert (25) and/or the threaded insert (13) is produced from a plastic which is provided with thermochromic masterbatches.

3. The hot-water bottle according to claim 1 or 2, **characterized in that** several floatable inserts (25) are provided, at least one of which forms the overheating indicator and one or more of which is (are) thermochromically set to lower temperature values.

4. The hot-water bottle according to any one of claims 1 to 3, **characterized in that** the plastic, the film and the finish, respectively, are provided with thermochromic masterbatches.

5. The hot-water bottle according to at least one of the preceding claims, **characterized in that** the overheating indicator (25 - 29) is set to a specific temperature in the range of from 60°C to 80°C, preferably at approximately 80°C.

## Revendications

1. Bouillotte (10) en matière plastique, comprenant un corps de bouillotte (11), un goulot (12) qui forme un entonnoir de remplissage, un bouchon (15) destiné à être vissé à étanchéité dans un insert taraudé (13) du goulot (12) ainsi qu'un indicateur de température sous forme d'un indicateur de surchauffement (25-29) pour le milieu liquide, de préférence l'eau, à verser dedans ou bien versé dedans, **caractérisée par le fait que** ledit indicateur de température est constitué par au moins une pièce insérée en ou avec une matière plastique thermochrome, qui est apte à flotter dans le milieu liquide du corps de bouillotte (11), ou par une zone dudit insert taraudé (13) dans le goulot (12), qui, sur son front annulaire (18, 19) dégagé axialement extérieur et/ou intérieur, est revêtue d'un film plastique thermochrome ou est pourvue d'un vernis thermochrome sur une zone dégagée.

2. Bouillotte selon la revendication 1, **caractérisée par le fait que** ladite pièce insérée (25) flottable et/ou ledit insert taraudé (13) est réalisé(e) à partir d'une matière plastique qui est pourvue de mélanges-maîtres thermochromes.

3. Bouillotte selon la revendication 1 ou 2, **caractérisée par le fait que** plusieurs pièces insérées (25) flottables sont prévues dont une au moins forme l'indicateur de surchauffement et dont une ou plusieurs sont réglées de manière thermochrome à des valeurs de température plus faibles.

4. Bouillotte selon l'une quelconque des revendications 1 à 3, **caractérisée par le fait que** la matière plastique, le film ou bien le vernis est pourvu(e) de mélanges-maîtres thermochromes.

5. Bouillotte selon l'une au moins des revendications précédentes, **caractérisée par le fait que** l'indicateur de surchauffement (25-29) est réglé à une température déterminée dans la plage allant de 60 °C à 80 °C, de préférence de 80 °C à peu près.
